# EUROPEAN PATENT APPLICATION

(11) **EP 3 536 233 A1**
(43) Date of publication of application: **11.09.2019**
(21) Application number: 18160526.2
(22) Date of filing: 07.03.2018
(51) Int. Cl.: A61B 5/0225, A61B 5/022, A61B 5/0295, A61B 5/00

(54) **BLOOD PRESSURE MEASUREMENT SYSTEM AND METHOD**

(71) Applicant: Koninklijke Philips N.V., 5656 AE Eindhoven (NL)
(72) Inventor: HILGERS, Achim, 5656 AE Eindhoven (NL); DELLIMORE, Kiran Hamilton, 5656 AE Eindhoven (NL)
(74) Representative: de Haan, Poul Erik

(57) **Abstract**

A blood pressure monitoring system is for mounting around a body limb or digit, in which an array of electroactive material actuators provides an inward force. One or more electroactive material actuators are identified which are best located for performing blood pressure monitoring and these are then used to apply a pressure. By performing pulse monitoring while controlling the identified one or more electroactive material actuators, the blood pressure may be determined.

## Description

### FIELD OF THE INVENTION

This invention relates to a blood pressure monitoring system, a blood pressure monitoring method and computer program product for performing the method.

### BACKGROUND OF THE INVENTION

There is increasing demand for unobtrusive health sensing systems. In particular, there is a shift from conventional hospital treatment towards unobtrusive vital signs sensor technologies, centered around the individual, to provide better information about the subject's general health.

Such vital signs monitor systems help to reduce treatment costs by disease prevention and enhance the quality of life. They may provide improved physiological data for physicians to analyze when attempting to diagnose a subject's general health condition. Vital signs monitoring typically includes monitoring one or more of the following physical parameters: heart rate, blood pressure, respiratory rate and core body temperature.

In the US about 30% of the adult population has a high blood pressure. Only about 52% of this population have their condition under control. Hypertension is a common health problem which has no obvious symptoms and may ultimately cause death, and is therefore often referred to as the "silent killer". Blood pressure generally rises with aging and the risk of becoming hypertensive in later life is considerable. About 66% of the people in age group 65-74 have a high blood pressure. Persistent hypertension is one of the key risk factors for strokes, heart failure and increased mortality.

The condition of the hypertensive patients can be improved by lifestyle changes, healthy dietary choices and medication. Particularly for high risk patients, continuous 24 hour blood pressure monitoring is very important and there is obviously a desire for systems which do not impede ordinary daily life activities.

Blood pressure is usually measured as two readings: systolic and diastolic pressure. Systolic pressure occurs in the arteries during the maximal contraction of the left ventricle of the heart. Diastolic pressure refers to the pressure in arteries when the heart muscle is resting between beats and refilling with blood. Normal blood pressure is considered to be 120/80 mmHg. A person is considered to be hypertensive when the blood pressure is above 130/90 or 140/90 mmHg (depending on the guidelines followed), and two stages of hypertension may be defined for increasing blood pressure levels, with hypertensive crisis defined when blood pressure reaches 180/110 mmHg. Note that for conversion of these values to metric equivalents, 760.0 mmHg is equal to 101.325 kPa (1 mm Hg = 133.32 Pa).

There are two main classes of method to monitor blood pressure.

For invasive direct blood pressure monitoring, the gold standard is by catheterization. A strain gauge in fluid is placed in direct contact with blood at any artery site. This method is only used when accurate continuous blood pressure monitoring is required in dynamic (clinical) circumstances. It is most commonly used in intensive care medicine and anesthesia to monitor blood pressure in real time.

For non-invasive indirect blood pressure monitoring, auscultatory and oscillometric methods are known. Both methods use a cuff placed around the arm, which is inflated at a pressure higher than the systolic pressure and then slowly deflated. The auscultatory method is based on listening to Korotkoff's sounds under the cuff (typically by the practitioner with a stethoscope), which appear when the cuff pressure is equal to the systolic pressure and disappear when the remaining pressure is equal to the diastolic pressure. The oscillometric method is based on measuring (electro-mechanically) pressure oscillations. These appear when the pressure in the cuff equals the systolic pressure, they are maximum at the mean pressure, and they disappear at the diastolic pressure.

In order to use a clamp or a cuff to determine the blood pressure (with help of suitable pressure detectors) a device is needed that can pressurize (parts of) the cuff or clamp to the required pressures and/or that can deliver the needed pressure variations.

Typically a compressor like device is used to deliver the required pressure. A blower like device is generally not suitable because the required cuff pressures of at least 100 mm Hg (approximately 13 kPa) above atmospheric pressure are demanding for such devices. A typical pump for this purpose for example operates at 6V with a current around 400mA, and for example results in noise up to 55 dB (at 30cm from the pump).

To avoid the need for an inflatable cuff, it has been proposed to perform blood pressure measurement using PPG sensors. For example, a pulse wave velocity may be measured using multiple PPG sensors. The pulse wave velocity (PWV) is the speed at which a pulse travels within the arteries. It is known that PWV is a measure of arterial stiffness and also correlates with blood pressure (either Mean Arterial Pressure (MAP), or Pulse Pressure (PP)). The PWV can be calculated from the pulse transit time (PTT; the time it takes for a blood pulse to travel from the heart to a certain location), or more generally from the pulse delay (PD; the difference in PTT of two different locations on the body). PWV measures may be made by measuring the time difference between the pulse arriving at one location on the body (such as the upper arm) and another location (such as the wrist). By detecting changes in blood volume using a PPG sensor, a cyclic signal corresponding to the pulse is obtained. PPG sensors, such as pulse oximeters, are thus commonly used to provide a measure of the pulse rate.

Another way to measure blood pressure (and without using any optical devices such as PPG sensors) is the tonometry method. This method is based on applying a controlled force orthogonally to the wall of a superficial artery against a bone. A force sensor measures the pressure at contact. This action on the superficial artery produces a local occlusion. There is no need for a surrounding cuff, but instead a supporting device is needed against which a pushing force can be applied. The applied force must be small in order not to completely close the artery, as in this case the blood pressure is not measured and there is a risk of ischemia. The applied force is varied to follow the pulse-pressure wave.

For correct measurements, the positioning of the tonometer over the center of the artery is very important as well as the force applied. The difference between correct and wrong placements is in the millimeter regime. When the sensor is placed incorrectly it will lead to a non-linear effect of the blood pressure on the sensor. As a result, the blood pressure will not be measured correctly. Tonometry is thus highly sensitive to motion, so it needs a continuous precise positioning of the sensor. Thus, tonometry is normally carried out with the subject at rest. The accuracy typically decreases rapidly over time and the device is not very comfortable for subjects during daily activities.

There are also cuff-based systems which make use of PPG measurement of the pulse. For example, it has been proposed in the article "Estimation of Blood Pressure Using Photoplethysmography on the Wrist" by SH Song et. al., Computers in Cardiology 2009; 36; 741-744 to combine a PPG measurement with an inflatable wrist cuff. Again, this requires a compressor like device to deliver the required pressure.

Finger-based cuffs with PPG measurement for determining blood pressure are also known and indeed commercially available, in which the applied pressure is controlled to maintain a constant PPG signal. These generally operate at a high pressure to provide partial occlusion of the blood flow.

All of these solutions have different problems associated with them so that remains a need for a low cost, low noise, compact and non-invasive way to measure blood pressure.

### SUMMARY OF THE INVENTION

According to examples in accordance with an aspect of the invention, there is provided a blood pressure monitoring system as defined in the independent claims.

The system includes a carrier for mounting at last partly against or around a body part such as e.g. neck, a limb (arm or leg), or digit/finger. The carrier has a first side on which actuators are arranged at different lateral positions. Thus, this positioning is such that when the carrier is mounted against or around the body part, the different ones of the plurality of actuators are located at different spatial or even angular positions with respect to the contours/rounding of the body part. With regard to the carrier the actuators thus face inwardly form the carrier.

Each electroactive material actuator is independently controllable/actuatable to provide a displacement and/or force in at least a direction away from the carrier. This means that at least a component of force generated by an actuator can be provided towards the body part when the carrier is positioned against or around the body part. They are thus actuatable inwardly in response to actuation and this actuation is meant for applying a force or pressure to the body part.

The system includes a pulse monitor for preforming pulse monitoring and outputting a pulse monitor signal.

A controller is provided capable of actuating the actuators and controlling the pulse monitor such that when the pulse monitor is monitoring, one or more of the plurality of actuators are actuated. Thus, during the pulse monitoring the one or more actuators that are actuated provide a pressure to the body part. The pulse monitor signal is thus obtained while or directly after actuation is performed.

The controller is capable of providing the pulse monitor signal, for example for further manipulation by the controller or other device. or for communication to a user by a user interface such as a display or sound provision device.

The system and/or controller may be adapted to determine a blood pressure from the pulse monitor signal and to output the determined blood pressure result for further manipulation or to a user using a user interface. The systems' controller can be adapted to this end. There may be a user interface such as a display to provide the pulse monitor signal and/or the blood pressure to a user. Alternatively or additionally, the system may have a data transfer unit that is wirelessly or wiredly connected to a remote control device such as a processor or computer in order to transfer the pulse monitor signal to the processor or remote control device which in turn is configured to determine the blood pressure from the transferred data and outputs the data and or the determined blood pressure to a user interface such as a display.

The pulse monitoring provides a signal which varies in dependence on the pressure applied by an actuation of one or more of the plurality of actuators. Thus, actuation may be employed to investigate the blood pressure. For example, much similar to some of the known methods described herein before, the applied pressure with actuation may be used/adjusted to make the pulse signal disappear in order to find or determine characteristics of the blood pressure. The reappearance of the pulse signal for example correlates to the systolic blood pressure. The diastolic blood pressure may be determined based on analysis of the pulse signal shape.

The disclosed arrangement makes use of a type of cuff formed of a carrier including electroactive material actuators to apply a pressure to the body part such as the limb or digit the carrier or cuff is attached to or around. The carrier as defined here is advantageous for use with a blood pressure monitoring system. It enables low noise operation and a compact or non-bulky device so that an even less invasive solution is provided for blood pressure monitoring. The independent controllability of the actuators means that only those actuators needed to provide pressure at the suitable location are used. This may provide improved comfort during use.

The controller can be further adapted to be able to identify one or more preferred electroactive material actuators of the plurality of actuators for use while performing the blood pressure monitoring. Preferred ones are those that for example result in improved signal stability or sensitivity during the measurements. Such preferred ones can be those with improved or best location with regard to a position on the body part. These actuators are identified during a calibration procedure which involves identifying the preferred (best to be used) actuators. In this way, the subsequent control of the actuators is more comfortable since a local pressure may be applied in the vicinity of the position (which may relate to that of an artery) rather than applying pressure around the whole body part.

The system may for example be for mounting around a finger, the wrist, the arm or the leg.

The system may be used as a wearable miniaturized system for continuous blood pressure measurement. It avoids the need for large volume air pumps and pressure variation may be made rapidly. Even a finger based system with a small cuff volume will need relatively powerful pumps and thus have a high electrical energy consumption to inflate the cuff and adapt the pressure. The system of the invention has low power consumption and is also able to operate with very low noise.

The pulse monitor preferably comprises a PPG sensor. This provides a low cost, compact, silent and low power monitoring method. However, other parameters may be measured which correlate to the pulse, such as the oxygen saturation level, SpO2.

The pressure which is applied by a given level of actuation of the actuators may be based on a factory calibration. To take account of the different possible digit or limb sizes, the actuators may be advanced until contact is made, and then the additional actuation correlates to the pressure applied.

The controller is for example adapted to identify the one or more electroactive material actuators by actuating the electroactive material actuators in a sequence and monitoring the effect on the pulse monitor signal. The sequence may for example comprise a circumferential sweep of the actuators, while monitoring the effect on the pulse monitor signal.

This may be performed once as a calibration step when the system is fitted. However, it may also be performed periodically, to take account of possible movement of the system over time, or different limb positions which may alter the mapping between actuator levels and the pressure in fact applied.

Each electroactive material actuator may comprise a beam that bows inwardly when actuated. This provides a simple way to provide an inward deflection. The beam may be fixed at one or both ends. It may also be in free suspended form kept in place within the carrier using a sliding holder or the like.

The plurality of electroactive material actuators may be arranged in an array of actuators. The plurality or array may for example comprises between 2 and 5, 2 and 10 or 2 and 20 or more actuators. The minimum number of 2 actuators may be increased to 5 or even 10 in the appropriate previously mentioned ranges of number of actuators. Preferably there are more than 4 actuators or even more than 8 actuators. The number of actuators may depend on the size of the carrier, for example depending on whether it is for a finger, wrist, arm or other body part. The resolution may be such that one actuator will be sufficient to apply a pressure to an artery location and the actuators are sufficiently close together that a pressure can be applied to any point.

The plurality of actuators or at least a part of them may be spaced along a length direction of the carrier, where the length direction is along a direction of bending (around a body part such as finger leg arm or other digit) when in use. Alternatively or additionally there may be actuators spaced apart in width direction (perpendicular to the length direction). The width direction may be a direction in which the carrier is not substantially bent when used against a body part such as a finger leg arm or other digit. The actuators are thus spaced apart laterally at the first side of the carrier (and this typically is the inside of the carrier when mounted around the body part).

The carrier may have a cylindrical shape at least when in use, i.e. mounted around or against the body part with the cylinder part having a cross-section with a radial dimension and a width dimension which is a direction in which the carrier is not substantially bent when mounted around a body part. The actuators may have a bending direction along which they bend upon their actuation. They may be attached to the carrier with this bending direction parallel to the width direction, perpendicular to the bending direction or angled (e.g. between 10 and 80 degrees or between 20 and 70 degrees) with regard to this bending direction. A combination of one or more of these directions is also possible. Having the bending direction not perpendicular to the width dimension of the carrier is advantageous when many actuators of considerable size need to be along side each other in a small circumferential section of the carrier when mounted.

The system may further comprise a respective force spreading unit attached to each electroactive material actuator. The force spreading unit may be have subunits each one of which is associated with at least one actuator or at most one actuator. Alternatively there may be one such unit that is flexible and is associated with all of the plurality of actuators. The actuators may provide a single point of contact where pressure is applied. The force spreading unit ensures that a pressure may be applied to all locations around the circumference of the limb or digit.

The identified one or more electroactive material actuators (i.e. those which are most suitable for blood pressure measurement) may comprise at least one electroactive material actuator identified as proximate an artery and at least one diametrically opposed electroactive material actuator to enhance a compression effect. In this way, a pressure may be applied more reliably to a desired location by implementing a pinching type compression.

The system may further comprise a pressure feedback system such as a pressure sensor for monitoring a pressure applied by the array of electroactive material actuators. This enables a mapping between the actuation level and the actual pressure applied so that the blood pressure levels may be determined more accurately. The pressure sensing also enables the point to be identified at which actuation of the actuators results in initial contact all around the limb or digit.

The pressure sensor may comprise a set of pressure sensing elements, with at least one associated with each electroactive material actuator. This enables local pressure sensing feedback.

The controller may be adapted to operate each electroactive material actuator to implement a pressure sensing function such that each electroactive material actuator functions as its own pressure sensing element. This approach takes advantage of the possible sensing functionality of some types of electroactive material actuator. The pressure sensing function may even be used to function as the pulse monitor by detecting skin palpitations.

The carrier may be flexible, e.g. elastic and stretchable. This may for example enable the carrier to be a contact fit with a variety of sizes of limb or digit so that the initial actuation of the actuators leads to an applied pressure. The carrier may also comprise a plurality of rigid parts that are movably connected to each other to form a chain so to speak.

The invention also provides a blood pressure monitoring method using a blood pressure monitoring system mounted around a body limb or digit, wherein the system comprises a carrier and an array of electroactive material actuators facing inwardly from the carrier, at different angular positions around the inside of the carrier,
wherein the method comprises:
independently controlling the electroactive material actuators to displace inwardly and monitoring a pulse thereby to identify one or more electroactive material actuators which are best located for performing blood pressure monitoring; and
performing pulse monitoring while applying a pressure using the identified one or more electroactive material actuators and thereby determine a blood pressure.

This method identifies a most suitable actuator or set of actuators before using that actuator or those actuators to perform blood pressure monitoring.

The pulse monitoring may comprise PPG sensing.

Identifying the one or more electroactive material actuators for example comprises actuating the electroactive material actuators in a sequence and monitoring the effect on a pulse monitoring signal.

The method may be implemented, at least in part, in computer software. The software is then capable of having the method performed by controlling the controller to have it perform the method using the system.
The method may comprise the steps of mounting the device on a body part and determining the blood pressure.

### BRIEF DESCRIPTION OF THE DRAWINGS

Examples of the invention will now be described in detail with reference to the accompanying schematic drawings, in which:
Figure 1 shows a known blood pressure monitoring system;
Figures 2A to 2C show examples of carriers with actuators in a blood pressure monitoring system;
Figure 3A shows the overall blood pressure monitoring system using the sensor part of Figure 2;
Figure 3B and 3C show examples of possible locations of the pressure sensor with respect to the actuators;
Figures 4A and 4B show the use of an additional contact element associated with each actuator;
Figures 5A and 5B show different orientations of the actuators with regard to the width directions of a carrier of a system.
Figure 6 shows a blood pressure monitoring method.

### DETAILED DESCRIPTION OF THE EMBODIMENTS

Embodiments of the invention and examples will be described with reference to the Figures. It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Disclosed is a blood pressure monitoring system for mounting around a body limb or digit, in which an array of electroactive material actuators provides an inward force, i.e. a force towards the body limb or digit. One or more electroactive material actuators are identified which are best located for use with performing blood pressure monitoring and these are then used to apply a pressure. By performing pulse monitoring while controlling the identified one or more electroactive material actuators, the blood pressure may be determined.

As mentioned above, a partly occlusive procedure to measure blood pressure using a finger cuff is known. This is for example known as the volume clamp method, and is described in N. M. L. Peter, "A review of methods for non-invasive and continuous blood pressure monitoring: Pulse transit time method is promising?", ScienceDirect, 2014.

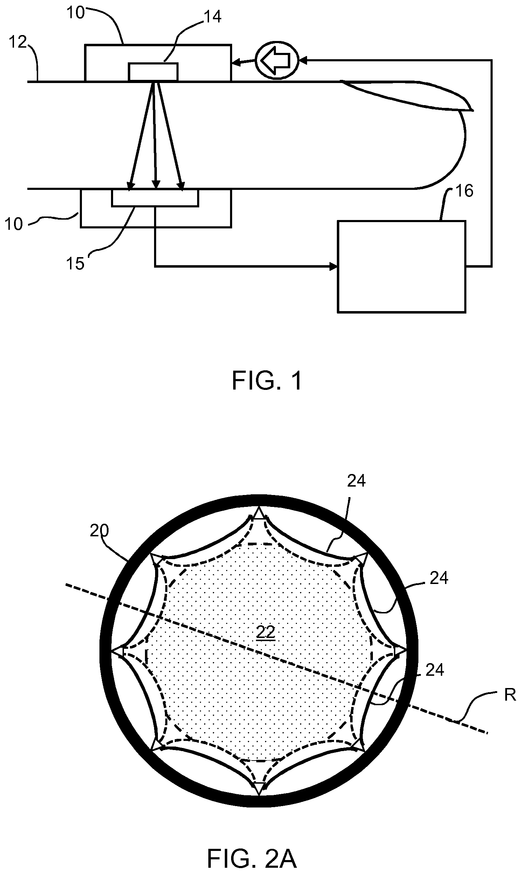

By way of example, Figure 1 shows a known arrangement of a cuff 10 placed around a finger 12 with a PPG sensor for measuring a pulse signal, comprising a light source 14 and detector 15. A controller 16 controls the application of pressure to the cuff to maintain a constant flow of blood under the cuff during each heartbeat. The PPG sensor 14, 15 does not provide blood pressure values but rather represents changes in the blood volume in the artery. Unfortunately, these volume changes cannot be transformed into pressure values because of the non-linearity of the elastic components of the arterial wall. Based on the changes in blood volume the pressure in the cuff is changing as well. The solution is to make the blood volume under the cuff constant. The blood pressure value can then be evaluated as the pressure value in the cuff. Therefore, the continuously changing pressure, which is applied from the outside of the finger, corresponds to the intra-arterial pressure and thus it is an instantaneous, continuous measure for arterial blood pressure.

Although the volume clamp method has disadvantages, in particular the reasonably high pressure that is applied to the tissue as well as the high frequency of pressure oscillations together create discomfort to the patient, it is an attractive solution for continuous blood pressure monitoring in certain applications, such as e.g. interventions. Thus several products which operating using this approach are available.

A proposed refinement to these systems is to apply a pre-pressure in the range of the systolic blood pressure and then to adapt this pressure by relatively small amounts based on evaluating a measured PPG signal at the same time. This avoids the need for application of a high pressure.

However, there remain problems associated with the use of a pressure cuff, as explained above.

Figure 2A shows an example of the physical sensor part of a blood pressure monitoring system in accordance with an example of the invention.

The sensor part comprises a carrier 20 for mounting around a body part such as a body limb or digit. The example shown is for mounting around a finger 22 and to this end has a radius in accordance with dimensions of a finger. The carrier may be configured to be removable for the body part. It may have an appropriate fastening means or device for that. It may also be part of a stretchable band that can be put around the body part. Other ways of attaching to carrier to the body part can be employed.

An array of electroactive material actuators 24 is provided, facing inwardly from the carrier 20, at different spatial and in this case also angular positions around the inside of the carrier. Each electroactive material actuator 24 is independently controllable to displace inwardly in response to an actuation signal. The solid lines represent the non-actuated configuration and the dashed lines represent the actuated configuration of an actuator. It can be seen in Figure 2A that in the context of the invention the term "inwardly" means that the actuators are arranged so as to be capable of providing a component of a force and/or a component of a displacement which is along the radial direction R and/or at least towards the finger enclosed. Only one example of a radial direction is shown for clarity.

Each electroactive material actuator 24 comprises a beam which is fixed at both ends (a double clamped arrangement) such that it bows inwardly when actuated as shown. This provides a simple way to provide an inward deflection of an actuator against the finger. The actuators for example comprise electroactive polymer actuators (of which examples will be described herein below), and they may comprise rectangular and/or elongated beams. Other shapes, such as square, circular, elliptical etc. are also possible. Other clamping arrangements or a clamping around the whole circumference (e.g. for a circular/elliptical actuator) are possible as well. Depending on the requirements, the actuators may have a pre-bend. The whole actuator array may be incorporated in a rigid, semi-rigid or stiff carrier 20 such as a ring, or a bandage-like configuration fixed with hook-and-loop fasteners. However, the carrier can also be flexible, but not stretchable

The example shows 8 actuators around the circumference of the carrier. More generally, there may be between 2 and 20 actuators or more spaced around the inside of the carrier. Preferably there are at least 4. The number of actuators will depend on the size of the system (and may be determined by use on a particular body part) and/or the pressure providing capability and/or precision required by the device. In this example the actuators together are designed to enable pressure to be applied all around the circumference, so that no matter where an artery is located in the finger, a pressure may be applied. This provides a freedom of mounting the device while being able to provide pressure to a preferred location without location. The resolution may be such that one actuator will be sufficient to apply a pressure to an artery location.

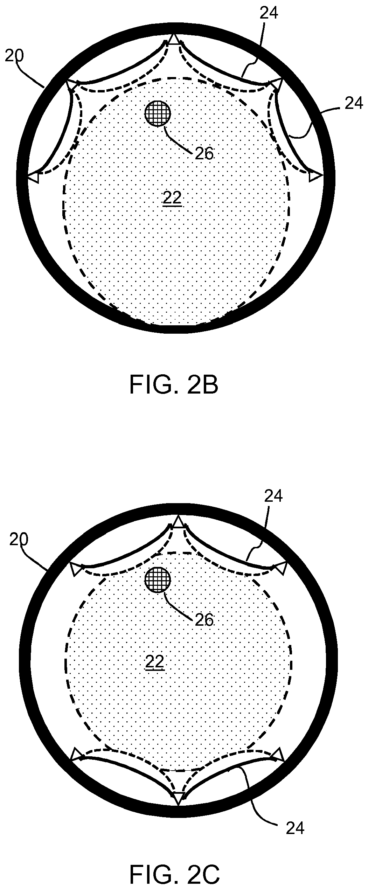

Although having actuators around the circumference may provide the best flexibility to the device for reasons indicated above and/or in terms of utility by freedom of orientation of the device around the body extremity upon its mounting, there need not be actuators located around the entire circumference of the carrier. This may reduce complexity of the device. Figure 2B and 2C show such examples where only part of the circumference has actuators. In the example of Figure 2B there is a set of abutting actuators (4 in total) covering half of the carriers 20 circumference which part is to be placed around a finger or other body part to be measured such that at least one artery 26 is within the circumference part covered by the actuators. A user, especially a medically trained one, can mount the device in such way with little effort. The actuators are abutting each other to cover an extended circumference area such that mounting is relatively easy and the best one or combination of actuators can be used/found for providing the pressure during monitoring. The part of the circumference not covered with actuators is to be placed against the body part to be measured. This part of the inside of the carrier may be provided with a lining (not shown in the figure) specifically shaped to contact a specific body part in order to improve the pressure distribution and/or comfort for a patient.

Figure 2C shows another example with actuators on opposite sides of a circumference of the carrier. Counterforces can now be provided from opposite sides, which may improve the monitoring. There may be multiple sets of actuators around a circumference and each of them can have at least 2 actuators or more abutting each other.

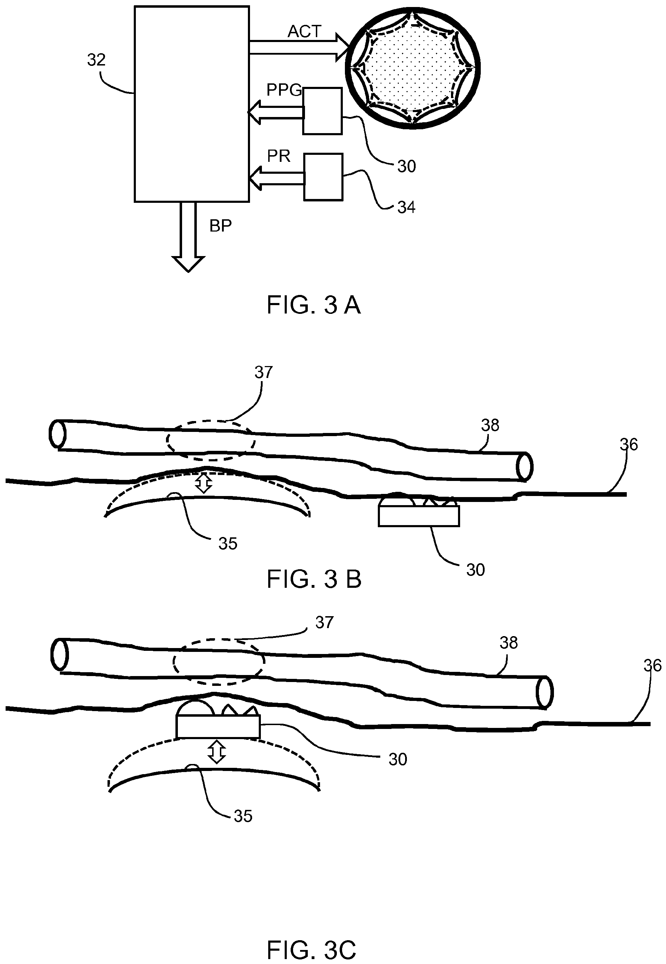

Figure 3 shows an example of an overall system. In addition to the actuator array as shown in e.g. Figure 2A or 2B or 2C, there is a pulse monitor 30 and a controller 32.

The pulse monitor may be a separate monitoring device to the array of actuators as shown in Figure 3B. In this Figure a disentangled pulse monitor 30 is shown to be pressed against a wall 36 of a body limb (e.g. finger). An artery 38 runs along the wall. An actuator 35 as described herein above is located near the sensor 30 and is capable of compressing the artery at location 37 to influence pulse characteristics at the location of the sensor 30. It may be a single sensor for providing a signal which is indicative of a pulse.

However, the sensor may instead be integrated into the structure of the array of actuators as for example shown in Figure 3C. In this figure the sensor 30 is mounted on the actuator 35 to press against the wall 36 of the finger to exert a pressure at the location 37 of the artery 38.

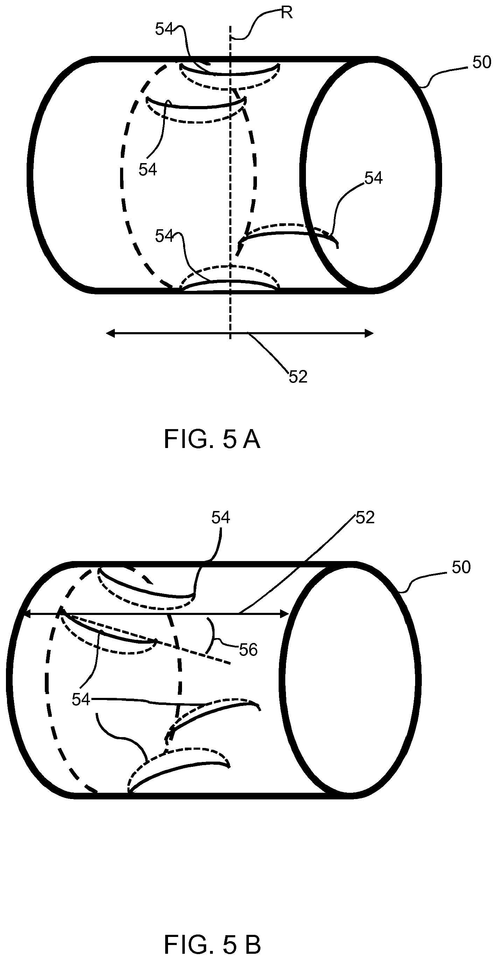

If the sensors are separate from the actuators, then they can be located between successive actuators around the circumference. Alternatively, they can be located next to the actuators in the width direction of the cylindrical carriers. See for example the discussion of Figure 5 in this respect.

There may be more than one pulse monitors in the overall system. Thus, a pulse monitor sensor may for example be provide on top of each actuator, or on top of a sub-set of the actuators. Likewise the multiple sensors may be separate from the actuators

The or each pulse monitor is preferably a PPG sensor which generates a PPG signal "PPG", although other sensors may be used such as an SpO2 sensor.

The controller generates actuation signals "ACT" for the actuators and outputs a blood pressure measurement or measurements "BP".

In preferred examples, the system also includes pressure sensing feedback. In one set of examples, the system then also comprises a pressure sensor 34 for monitoring a pressure applied by the array of electroactive material actuators and providing a pressure feedback signal "PR". This enables a mapping between the actuation level and the actual pressure applied. The pressure sensing may also enable the actuation level to be identified at which initial contact is made all around the limb or digit.

The pressure sensor 34 may comprise a set of pressure sensing elements, with at least one associated with each electroactive material actuator. The pressure sensor may be integrated into or on the physical structure of each actuator. This enables local pressure sensing feedback. The pressure sensing function may instead be implemented by the actuators themselves if they have both actuation and sensing functionality.

The controller performs various functions as part of an overall control algorithm. It is used to actuate the array of electroactive material actuators, but with individual control of the actuators. In particular, there is a calibration cycle in which one or more electroactive material actuators are identified which are best located for performing blood pressure monitoring. Pulse monitoring using the pulse monitor is then carried out while controlling the identified one or more electroactive material actuators to apply a pressure, and from the pulse monitor signals the blood pressure is obtained.

The independent controllability of the actuators means that only those actuators needed to provide pressure at the suitable location are used. A comfortable system results with pressure applied only where needed to implement the blood pressure monitoring.

The use of actuators avoids the need for an inflatable cuff. However, it is noted that the actuators can be combined with an existing inflatable cuff. The cuff may be used to provide a default pressure level, and the actuators then take over control. Alternatively, a pressure cuff may be used as a pressure sensing arrangement. For example, a set of small (closed) air cuffs may be provided around the actuator array. The deformation of each actuator results in a pressure increase towards the tissue but at the same time in the small closed cuff. This for example allows known air pressure sensing methods to be used for pressure sensing feedback, for example by having an air pressure sensor associated with each small cuff. Such pressure sensing may be used both for measuring the static pressure and detecting blood oscillations.

As mentioned above, in one preferred set of examples, the pressure feedback is achieved by the actuators being able to operate in a sensing mode.

One approach is to combine a DC actuation signal with a high frequency superposed AC signal for sensing. This approach is described in detail in WO 2017/036695. WO 2017/037117 discloses another example of a sensor and actuator which enables pressure sensing as well as temperature sensing.

During actuation, the actuators generate a well-defined force and/or deformation as a function of the applied actuation signal, which is a voltage if the actuator is a voltage controlled actuator. The signal may also be of different nature as determined by the type of actuator used. This deformation can be assumed to be known from a calibration process, even at different loads (the different actuation load is equivalent to generating different pressures to the tissue).

During actuation, an impedance change of the actuators can also be measured as the sensing function. This impedance in combination with the applied voltage allows the (quasi-static) pressure generated towards the tissue to be determined.

In this quasi-static mode, the pressure variation caused by the puled blood flow can also be detected by using the actuator in sensor mode only. The palpitation (of the skin) will also slightly deform the actuator and this will change the impedance.

Thus both global slow pressure adjustment and local fast pulse pressure variations can be detected. The local variations can be used to generate the desired pulse signal and the actuators may thus also function as the pulse monitor. Alternatively, the detected pulse signal can be used as additional sensing information to the PPG signal.

The application of pressure and monitoring of pulse strength or signal shape may follow known approaches, for example as outlined in "Estimation of Blood Pressure Using Photoplethysmography on the Wrist" as referenced above.

The applied pressure may for example be increased to make the pulse monitor signal disappear. The reappearance of the pulse signal for example correlates to the systolic blood pressure. The diastolic blood pressure may be determined based on analysis of the pulse signal shape.

The control algorithm relies on the actuation level of the actuator and the sensed pressure in order to control the pressure exerted by each of the actuators. When each actuator also acts as sensor once a steady state is reached, it is possible to determine the local pressure applied by each actuator and then adjust the level of actuation to obtain an accurate and reliable PPG measurement while ensuring patient comfort.

The control algorithm therefore functions differently from a cuff, since in a cuff the pressure cannot be locally varied as can be achieved with a single actuator.

In the non-actuated state of the system, the actuator array can be easily put over the finger, since the actuator array is not expanded. After applying the array to the finger, it can be activated, i.e. a voltage can be applied to the individual actuator elements of the array, and accordingly each actuator starts to deflect. Due to the double-clamped arrangement, the only moving direction of the actuators is towards the finger. Therefore, with increasing voltage and resulting advancing movement, the tissue will be compressed progressively until the required pressure is reached, and the voltage applied to the actuators is then not increased any further.

The arrangement of actuators shown in Figure 2 may result in a spot-like compression around the tissue. However, a more uniform pressure distribution may be of benefit.

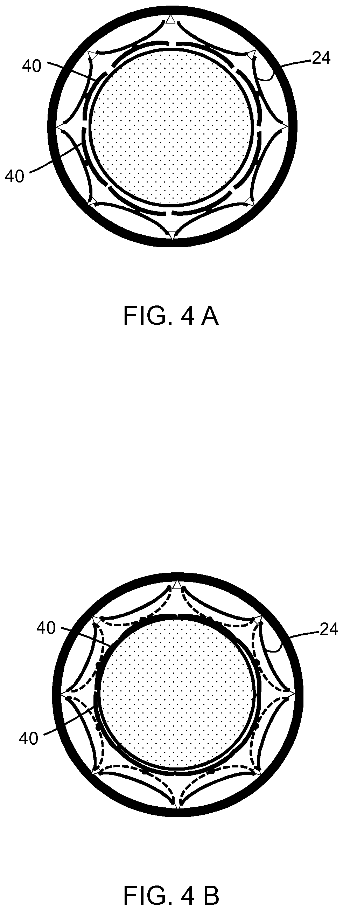

Figures 4A and 4B show the use of an additional contact element 40 mechanically in contact with an associated actuator 24, either in a stable form (e.g. glued) or in a flexible way (e.g. via a pivot element). These contact elements 40 function as force spreading units. The force spreading units 40 ensure that a pressure may be applied to all locations around the circumference of the limb or digit. The force spreading units 40 have a design which fits the tissue to be compressed, for example a set of concave elements which together generally match the cross sectional shape of the finger. In the Figures 4A and 4B, the units 40 are separate from each other giving independent freedom to operate. However, they need not all be independent. The multiple units 40 or abutting sub-sets of the multiple units may be combined in one unit. Preferably such combined unit is flexible. All units may be in one single flexible unit around the circumference of the carrier. The flexibility can provide pseudo independent functionality. Flexible materials can include units having rubber parts or the like. If in single unit form, it may be closed or open on one location. The latter configuration as are the multiple units 40 can be used with a carrier that has an opening with fastener for easy mounting around a body part.

Figure 4A shows the non-activated state of the actuators and Figure 4B shows the activated state in which the force spreading units provide a more uniform contact around the finger. The force spreading units may be made from a solid and rigid material but they may also made from a softer or semi-rigid material in order to be slightly flexible and even better fit around the tissue to be compressed. In particular, at the ends of the force spreading units there may be a soft region so that they may also overlap and thus enable fitting to different sizes of finger.

In the examples the carrier may have a length direction along a bending direction and a width direction perpendicular to that. For example the length direction is along the circumference of a carrier 20 in the plane of drawing of Figures 2 and 4. The width of a carrier is then perpendicular to the plane of drawing of Figures 2 or 4.

In the examples above the actuators 24 are laterally displaced along the length direction and the beam shaped actuators are oriented with their bending direction along the length direction. This may be different in that the beams may be parallel to the width direction. Furthermore, and this is not shown in Figures 2 and 4, there may be multiple actuators along the width direction. In the examples hereinbefore, the actuators have been shown to be arranged parallel to the circumference of the carrier, i.e. transverse to the width direction of the carrier. This need not be the case per se. In alternative examples one or more of the actuators are oriented partially in a longitudinal direction.

The carrier in general may have the shape of a cylinder or part of a cylinder (not necessarily with a circular or round cross-section) when in use. It thus can have a cross-sectional dimension (radius if circular cross-section) and a width. In such configuration, the width of the carrier is along the direction 52 in Figure 5 for example. and the length direction of the carrier (not indicated) is along the direction of bending or perpendicular to the direction 52.

When mounted to a body part, the width direction will thus be substantially parallel to the extension of the body part, e.g. length direction of a finger

Figure 5 shows a cylindrical carrier 50 with substantially circular cross-section. The radial R is indicated as is the width of the cylinder 52. In this example there are two pairs of oppositely mounted actuators 54 mounted around subsections of the inner wall of the cylinder. Note however that the actuators are now substantially parallel to the width direction with their length direction (along which they bend upon actuation) as opposed to the examples hereinbefore. They may also be oriented with an angle 56 towards the width direction that is not 0 or 90 degrees, but for example 30, 45 or 60 degrees. This is schematically sown in Figure 5B.

The advantage of mounting as in figures 5A and 5B may be that if many actuators need to be spaced within a small circumference area, this can be done without loss or with only limited loss of force and/or displacement. After all, in such configurations, the actuators need not be reduced in length size, which would have been the case if an increasing number of actuators oriented in the way as for example shown in Figure 2A was to be placed within a fixed circumference part. Another example would be that arteries that run in different directions under the skin through the body parts can be better targeted. To this end a combination of different orientations can be beneficial too.

With all the examples, the actuators may be distributed along the width. Thus there may be more than one location along the width direction of the carrier that has an array of actuators as in Figure 2 or 5A. This further provides a better chance of covering an artery for good measurement. Alternatively if there are more than one pulse sensor there may be more arteries to measure on. Comparison of the results may be performed to get a better estimation of blood pressure.

Again in this configuration the number and location of the actuators can be as described herein before with all their advantages.

During the control algorithm outlined above, instead of adapting the uniform pressure around the tissue, the pressure at one or more dedicated actuators is adjusted. Thus, only one or more actuators will change their actuation level, causing a spot-wise variation of the applied pressure to the tissue. This may be of importance if the pressure only above an artery needs to be adapted, while the tissue around the artery may be constantly compressed, which may improve the sensing signal but also may be more comfortable for the user.

A calibration routine to identify those actuators comprises a cyclic measurement of the effect of changing the pressure locally around the digit or limb, so that the subsequent signal collection phase involves only adapting the pressure at the point(s) where the best sensing signal to noise ratio is achieved. This may be exactly above an artery. In addition, one or more diametrically opposite actuators may also be addressed (and actuated with the same actuation level) in order to strengthen the compression effect.

This cyclic calibration may be performed at diastolic blood pressure (∼80 mmHg) or lower in order to ensure patient comfort.

There may also be a factory calibration by which the actuator activation level is mapped to a pressure level. To take account of the different possible digit or limb sizes, the actuators may be advanced until contact is made, and then the additional actuation correlates to the pressure applied using this calibration data.

The overall operation of the system (without relying on additional pressure sensing feedback) may be carried out in the following way:
Initially there is no contact between the actuators and the tissue.

There is then slow slowly actuating of the actuators, with parallel measurement of the the impedance of the actuators as the feedback sensing function.

In an unloaded configuration the impedance change as a function of actuation level can be assumed to be known because of calibration of the device prior to the application (with the calibration at an unloaded situation).

When an abrupt change in the impedance is detected or any deviation from the calibration data, the actuator can be determined to be in contact with the tissue.

From this point on, a different loaded actuator calibration look up table can be used to generate the pre-pressure required in the application. Once the pre-pressure has been reached (which is a quasi static pressure), the PPG signal can be determined.

As also mentioned above, separate pressure sensing feedback may be provided for example to make the method more accurate. Pressure sensors may also directly measure the blood pressure. These pressure sensors may be implemented into the actuator array configuration. For example, at least one, but preferably each, actuator may be equipped with a pressure sensor such as a piezoelectric sensor, PVDF foil, etc.

As explained above the pressure sensing feedback may thus be based on separate pressure sensors, an inflated cuff which is used to detect pressure, or the sensing functionality of the actuators themselves.

When a pressure is applied to the tissue, this pressure can be measured via the sensor. Accordingly a well-defined pressure can be applied and also changes in oscillations in the blood vessels can be detected while varying the applied pressure thereby implementing an oscillometric method. This method for example involves increasing the pressure above the systolic pressure, followed by a decrease down to below the diastolic pressure, or alternatively starting below the diastolic pressure and increasing up to the systolic pressure.

Note that the apparatus also enables a tonometry method or a volume clamp procedure to be realized. The pressure sensing may also make use of the intrinsic pressure sensing function of the actuators, in particular electroactive polymer actuators.

The system also enables compensation for motion artefacts for example caused by twisting movements of the array of actuators in relation to the skin surface, or motions of the finger (e.g. finger kinking), which will have an impact on the size and shape of the finger at the location of the actuator array and the mechanical properties of the tissue (due to stretching/compressing). The position of the actuator array may also move along the finger slightly. Therefore the actuator array may thus adapt the applied pressure so that pressure changes caused by such motions will be compensated.

Differences in finger tissue geometry may also arise due to swelling resulting from elevated ambient temperature conditions, increased salt consumption, or inflammation caused by health conditions such as osteoarthritis.

In some cases, a rigid array of actuators may cause discomfort and/or sub-optimal finger contact such as too high or low contact pressure. The carrier 20 may be fabricated from a soft, elastic, flexible, stretchable skin conforming material, such as silicone rubber, which can easily accommodate and compensate for natural changes in tissue geometry. This offers several additional advantages. There may be a lowering of the pressure needed by the array of actuators for a suitable contact pressure to be maintained compared to a solid implementation. A pre-pressure (i.e., a base pressure) may be applied by the carrier to the finger tissue which is close to the diastolic pressure, thereby enabling the actuator array to be used only for generating pressure variations. Finally, slippage of the actuator array along the finger while being worn by a user may be reduced since the silicone rubber material may be textured with a high contact friction, therefore enabling it to adhere to skin in a more conformal manner than a solid or semi-rigid material. This may be helpful in reducing some motion artefacts.

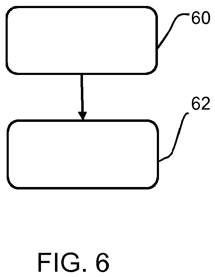

Figure 6 shows a blood pressure monitoring method using the blood pressure monitoring system described above. The method comprises:
in step 60, independently controlling the electroactive material actuators to displace inwardly and monitoring a pulse thereby to identify one or more electroactive material actuators which are best located for performing blood pressure monitoring; and
in step 62, performing pulse monitoring while applying a pressure using the identified one or more electroactive material actuators and thereby determine a blood pressure.

In all examples, the electroactive material actuator is typically based on an electroactive polymer material, although the invention can in fact be used for devices based on other kinds of EAM material. Such other EAM materials are known in the art and the person skilled in the art will know where to find them and how to apply them. A number of options will be described herein below. EAMs can work as sensors or actuators and can easily be manufactured into various shapes allowing easy integration into a large variety of systems.

A common sub-division of EAM devices is into field-driven and current or charge (ion) driven EAMs. Field-driven EAMs are actuated by an electric field through direct electromechanical coupling, while the actuation mechanism for current or charge driven EAMs involves the diffusion of ions. The latter mechanism is more often found in the corresponding organic EAMs such as EAPs. While field driven EAMs generally are driven with voltage signals and require corresponding voltage drivers/controllers, current driven EAMs generally are driven with current or charge signals sometimes requiring current drivers. Both classes of materials have multiple family members, each having their own advantages and disadvantages.

Field driven EAMs can be organic or inorganic materials and if organic can be single molecule, oligomeric or polymeric. For the current invention they are preferably organic and then also oligomeric or even polymeric. The organic materials and especially polymers are an emerging class of materials of growing interest as they combine the actuation properties with material properties such as light weight, cheap manufacture and easy processing.

The field driven EAMs and thus also EAPs are generally piezoelectric and possibly ferroelectric and thus comprise a spontaneous permanent polarization (dipole moment). Alternatively, they are electrostrictive and thus comprise only a polarization (dipole moment) when driven, but not when not driven. Alternatively they are dielectric relaxor materials. Such polymers include, but are not limited to, the sub-classes: piezoelectric polymers, ferroelectric polymers, electrostrictive polymers, relaxor ferroelectric polymers (such as PVDF based relaxor polymers or polyurethanes), dielectric elastomers, liquid crystal elastomers. Other examples include electrostrictive graft polymers, electrostrictive paper, electrets, electroviscoelastic elastomers and liquid crystal elastomers.

The lack of a spontaneous polarization means that electrostrictive polymers display little or no hysteretic loss even at very high frequencies of operation. The advantages are however gained at the expense of temperature stability. Relaxors operate best in situations where the temperature can be stabilized to within approximately 10 °C. This may seem extremely limiting at first glance, but given that electrostrictors excel at high frequencies and very low driving fields, then the applications tend to be in specialized micro actuators. Temperature stabilization of such small devices is relatively simple and often presents only a minor problem in the overall design and development process.

Relaxor ferroelectric materials can have an electrostrictive constant that is high enough for good practical use, i.e. advantageous for simultaneous sensing and actuation functions. Relaxor ferroelectric materials are non-ferroelectric when zero driving field (i.e. voltage) is applied to them, but become ferroelectric during driving. Hence there is no electromechanical coupling present in the material at non-driving. The electromechanical coupling becomes non-zero when a drive signal is applied and can be measured through applying the small amplitude high frequency signal on top of the drive signal. Relaxor ferroelectric materials, moreover, benefit from a unique combination of high electromechanical coupling at non-zero drive signal and good actuation characteristics.

The most commonly used examples of inorganic relaxor ferroelectric materials are: lead magnesium niobate (PMN), lead magnesium niobate-lead titanate (PMN-PT) and lead lanthanum zirconate titanate (PLZT). But others are known in the art.

PVDF based relaxor ferroelectric based polymers show spontaneous electric polarization and they can be pre-strained for improved performance in the strained direction. They can be any one chosen from the group of materials herein below.

Polyvinylidene fluoride (PVDF), Polyvinylidene fluoride - trifluoroethylene (PVDF-TrFE), Polyvinylidene fluoride - trifluoroethylene - chlorofluoroethylene (PVDF-TrFE-CFE), Polyvinylidene fluoride - trifluoroethylene - chlorotrifluoroethylene) (PVDF-TrFE-CTFE), Polyvinylidene fluoride- hexafluoropropylene (PVDF - HFP), polyurethanes or blends thereof.

The sub-class dielectric elastomers includes, but is not limited to: acrylates, polyurethanes, silicones.

Examples of ionic-driven EAPs are conjugated polymers, carbon nanotube (CNT) polymer composites and Ionic Polymer Metal Composites (IPMC).

The sub-class conjugated polymers includes, but is not limited to:
polypyrrole, poly-3,4-ethylenedioxythiophene, poly(p-phenylene sulfide), polyanilines.

The materials above can be implanted as pure materials or as materials suspended in matrix materials. Matrix materials can comprise polymers.

To any actuation structure comprising EAM material, additional passive layers may be provided for influencing the behavior of the EAM layer in response to an applied drive signal.

The actuation arrangement or structure of an EAM device can have one or more electrodes for providing the control signal or drive signal to at least a part of the electroactive material. Preferably the arrangement comprises two electrodes. The EAM layer may be sandwiched between two or more electrodes. This sandwiching is needed for an actuator arrangement that comprises an elastomeric dielectric material, as its actuation is among others due to compressive force exerted by the electrodes attracting each other due to a drive signal. The two or more electrodes can also be embedded in the elastomeric dielectric material. Electrodes can be patterned or not.

It is also possible to provide an electrode layer on one side only for example using interdigitated comb electrodes.

A substrate can be part of the actuation arrangement. It can be attached to the ensemble of EAP and electrodes between the electrodes or to one of the electrodes on the outside.

The electrodes may be stretchable so that they follow the deformation of the EAM material layer. This is especially advantageous for EAP materials. Materials suitable for the electrodes are also known, and may for example be selected from the group consisting of thin metal films, such as gold, copper, or aluminum or organic conductors such as carbon black, carbon nanotubes, graphene, poly-aniline (PANI), poly(3,4-ethylenedioxythiophene) (PEDOT), e.g. poly(3,4-ethylenedioxythiophene) poly(styrenesulfonate) (PEDOT:PSS). Metalized polyester films may also be used, such as metalized polyethylene terephthalate (PET), for example using an aluminum coating.

The materials for the different layers will be selected for example taking account of the elastic moduli (Young's moduli) of the different layers.

Additional layers to those discussed above may be used to adapt the electrical or mechanical behavior of the device, such as additional polymer layers.

As mentioned above, an electroactive polymer structure may be used both for actuation and for sensing. The most prominent sensing mechanisms are based on force measurements and strain detection. Dielectric elastomers, for example, can be easily stretched by an external force. By putting a low voltage on the sensor, the strain can be measured as a function of voltage (the voltage is a function of the area).

Another way of sensing with field driven systems is measuring the capacitance-change directly or measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. O₂, NO₂), making CNTs usable as gas detectors.

The preferred implementation of the invention makes use of PPG sensing. A pulse oximeter is a common example of a PPG-based sensor. The purpose of pulse oximetry is to monitor the oxygen saturation of a patient's blood. While the purpose of such a sensor is to obtain a measure of blood oxygen saturation, it also detects changes in blood volume in the skin, and thereby performs PPG sensing. By detecting changes in blood volume, a cyclic signal corresponding to the pulse is obtained. PPG sensors, such as pulse oximeters, are thus commonly used to provide a measure of the pulse rate.

A PPG sensor contains at least one LED, and one light sensor. The LED and sensor are placed such that the LED directs light into the skin of the user, which is reflected or transmitted, and detected by the sensor. The amount of reflected/transmitted light is determined by, amongst others, the perfusion of blood within the skin.

The PPG system for example includes a red LED, a near-infrared LED, and a photodetector diode. The LEDs emit light at different wavelengths, which light is diffused through the vascular bed of the patient's skin and received by the photodetector diode. The changing absorbance at each of the wavelengths is measured, allowing the sensor to determine the absorbance due to the pulsing arterial blood alone, excluding venous blood, skin, bone, muscle, and fat for example. The resulting PPG signal may then be analyzed.

Other simpler versions of a system for obtaining PPG data may be used, including a version with a single light source of one or more wavelengths. The absorption or reflectance of the light is modulated by the pulsatile arterial blood volume and detected using a photodetector device.

In transmissive pulse oximetry, a sensor device is placed on a thin part of the patient's body. Reflectance pulse oximetry may be used as an alternative to transmissive pulse oximetry. This method does not require a thin section of the person's body and is therefore well suited to more universal application.

A basic design of a PPG sensor for example has a certain light output frequency (e.g. 128Hz) with which the light source is pulsed. A sampling frequency of the optical sensor is higher, for example 256Hz so that it measures during light source activation and between light source activations. This allows the system to distinguish between the emitted light from the LED and the ambient light, and thereby filter out the ambient light from the signal received during a light source pulse.

As discussed above, a controller performs the data processing. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

It should be understood that the detailed description and specific examples, while indicating exemplary embodiments of the apparatus, systems and methods, are intended for purposes of illustration only and are not intended to limit the scope of the invention.

## Claims

1. A blood pressure monitoring system, comprising:
- a carrier (20) having a first side, the carrier being configured for mounting at least partly against or around a body part (22) with its first side facing the body part;
- a plurality of electroactive material actuators (24) arranged at different lateral positions at the first side of the carrier, each one of the plurality of electroactive material actuators being independently actuatable to provide a displacement and/or force at least in a direction away from the carrier;
- a pulse monitor (30) for performing pulse monitoring and outputting a pulse monitor signal; and
- a controller (32) adapted to:
- actuate one or more of the plurality of electroactive material actuators (24);
- while or directly after actuating the one or more of the plurality of electroactive material actuators control the pulse monitor to perform pulse monitoring; and
- determine the pulse monitor signal obtained with the pulse monitoring.

2. A blood pressure monitoring system as claimed in claim 1, wherein the system or controller is further adapted to determine a blood pressure from the pulse monitor signal.

3. A blood pressure monitoring system as claimed in claim 1 or 2, wherein the controller is further adapted to:
- identify one or more preferred electroactive material actuators of the plurality of electroactive material actuators for use with performing the pulse monitoring;
- perform the pulse monitoring while using the identified one or more electroactive material actuators.

4. A system as claimed in any of the previous claims, wherein the pulse monitor (30) comprises a PPG sensor.

5. A system as claimed in any preceding claim, wherein the controller (32) is adapted to identify the one or more electroactive material actuators by actuating the electroactive material actuators in a sequence and monitoring the effect on the pulse monitor signal.

6. A system as claimed in any preceding claim, wherein each electroactive material actuator (24) comprises a beam which bows upon actuation.

7. A system as claimed in any preceding claim, wherein the plurality of electroactive material actuators comprises between 2 and 20 actuators spaced apart laterally at the first side of the carrier.

8. A system as claimed in any preceding claim, further comprising a respective force spreading unit (40) attached to each electroactive material actuator (24).

9. A system as claimed in any preceding claim, wherein the identified one or more electroactive material actuators comprise at least one electroactive material actuator identified as proximate an artery and at least one diametrically opposed electroactive material actuator to enhance a compression effect.

10. A system as claimed in any preceding claim, further comprising a pressure sensor (34) for monitoring a pressure applied by the array of electroactive material actuators.

11. A system as claimed in claim 10, wherein the pressure sensor comprises a set of pressure sensing elements, with at least one associated with each electroactive material actuator.

12. A system as claimed in claim 11, wherein the controller is adapted to operate each electroactive material actuator to implement a pressure sensing function such that each electroactive material actuator functions as its own pressure sensing element.

13. A system as claimed in any preceding claim, wherein the carrier (20) is flexible.

14. A method of controlling a blood pressure monitoring system comprising:
- a carrier (20) having a first side, the carrier being configured for mounting at least partly against or around a body part (22) with its first side facing the body part;
- a plurality of electroactive material actuators (24) arranged at different lateral positions at the first side of the carrier, each one of the plurality of electroactive material actuators being independently actuatable to provide a displacement and/or force at least in a direction away from the carrier;
- a pulse monitor (30) for performing pulse monitoring and outputting a pulse monitor signal,
wherein the method comprises:
- actuating one or more of the plurality of electroactive material actuators (24);
- during or directly after actuating the one or more of the plurality of electroactive material actuators, control the pulse monitor to perform pulse monitoring; and
- determine the pulse monitor signal obtained with the pulse monitoring.

15. A method as claimed in claim 14, comprising:
(50) independently actuating one or more of a plurality of electroactive material actuators and performing pulse monitoring thereby to identify one or more actuators which are best located for performing pulse monitoring; and
(52) performing the pulse monitoring for determining the pulse monitoring signal while actuating one or more of the identified one or more actuators.

16. A method as claimed in claim 14, wherein identifying the one or more electroactive material actuators comprising actuating the electroactive material actuators in a sequence and monitoring the effect on a pulse monitoring signal.

17. A method as claimed in any of the claims 14 to 16, comprising:
- mounting the carrier against or around a body part;
- performing the steps as defined in any of the claims 14 to 16; and
- determining a blood pressure form the pulse monitoring signal.

18. A computer program comprising computer program code means which is adapted, when said program is run on a computer, to implement the method of controlling of the system according to any one of claims 14 to 17.
